# EUROPEAN PATENT SPECIFICATION

(11) **EP 4 173 574 B1**
(45) Date of publication and mention of the grant of the patent: **19.02.2025**
(21) Application number: 22790189.9
(22) Date of filing: 17.03.2022
(51) Int. Cl.: A61B 5/11, A61B 5/18, G02B 27/01, G06F 3/01, G16H 50/30, G16H 20/70, G06T 19/00

(54) **DEVICE FOR ESTIMATING COGNITIVE ABILITY, AND PROGRAM**
VORRICHTUNG ZUR SCHÄTZUNG DER KOGNITIVEN FÄHIGKEIT, UND PROGRAMM
DISPOSITIF D'ESTIMATION DE LA CAPACITÉ COGNITIVE, ET PROGRAMME

(30) Priority: 10.09.2021 JP 2021148075; 24.02.2022 JP 2022027232
(43) Date of publication of application: 03.05.2023
(73) Proprietor: mediVR, Inc., Toyonaka City, Osaka 561-0872 (JP)
(72) Inventor: HARA, Masahiko, Osaka 5610872 (JP)
(74) Representative: TBK
(86) International application number: PCT/JP2022/012211
(87) International publication number: WO 2023/037619

(56) References cited:
- EP-A1- 3 777 810
- WO-A1-2020/152779
- JP-A- 2007 292 908
- JP-A- 2010 144 701
- JP-A- 2020 110 561
- JP-A- 2021 511 591
- JP-B1- 6 898 675
- JP-B2- 6 914 896
- US-A1- 2012 150 545
- US-A1- 2019 247 719
- US-A1- 2022 076 803

## Description

### TECHNICAL FIELD

The present invention relates to a cognitive ability estimation apparatus, and a program.

### BACKGROUND ART

In the above technical field, patent literature 1 discloses a technique of supporting rehabilitation of a patient.

### CITATION LIST

### PATENT LITERATURE

Patent literature 1: Japanese Patent Laid-Open No. 2015-228957 EP 3 777 810 A1 discloses a rehabilitation support apparatus that provides rehabilitation comfortable for a user includes a detector that detects a direction of a head of a user wearing a head mounted display, a first display controller that generates, in a virtual space, a rehabilitation target object to be visually recognized by the user and displays the target object on the head mounted display in accordance with the direction of the head of the user detected by the detector, and a second display controller that displays, on the head mounted display, a notification image used to notify the user of a position of the target object.

WO 2020/152779 A1 discloses a rehabilitation system for performing rehabilitation of higher brain dysfunction, the rehabilitation system comprising: an image processing device which executes an application for presenting a patient with a problem for rehabilitation based on images using virtual reality, augmented reality, or mixed reality, and stores, as rehabilitation history information, the patient's problem solving history; a practitioner-side terminal which receives the rehabilitation history information from the image processing device; a server which stores the rehabilitation history information sent from the practitioner-side terminal; and a doctor-side terminal which receives rehabilitation history information from the server and displays the implementation status of the rehabilitation of the patient on the basis of the rehabilitation history information.

US 2019/247719 A1 discloses a system configured to perform active target updating according to a rehabilitation action of a user, in which a first rehabilitation action of the user is detected, and an avatar image that moves in accordance with the detected first rehabilitation action and a target image representing a target of the first rehabilitation action are displayed. The rehabilitation ability of the user is evaluated by comparing the first rehabilitation action and a target position represented by the target image, and the target position is updated in accordance with an evaluation result. Furthermore, a second rehabilitation action of the user during the first rehabilitation action is detected, and the rehabilitation ability is evaluated based on both the first rehabilitation action and the second rehabilitation action. In addition, when at least a predetermined evaluation is made only for the first rehabilitation action, the rehabilitation ability is evaluated based on both the first rehabilitation action and the second rehabilitation action.

US 2012/150545 A1 discloses a battery of three or more sensory and cognitive challenge tasks which actively or dynamically challenge the brain to monitor its state for assessment of injury, disease, or compound effect, among others. The system analyzes and assesses a personalized biometric brain health signature by integrating the use of electroencephalography (EEG), somato-sensory, neuropsychological, and/or cognitive stimulation, and novel signal processing and display. The system also provides for early detection of dementia, including Alzheimer's disease (AD), vascular dementia (VAD), mixed dementia (AD and VAD), MCI, and other dementia-type disorders, as well as brain injury states such as mild Traumatic Brain Injury and can provide some or all of the following improvements over conventional systems and methods, including: (1) Increased sensitivity, specificity, and overall accuracy; (2) early detection of disease and injury; and (3) enhanced portability with remote data acquisition capability.

### SUMMARY OF THE INVENTION

### TECHNICAL PROBLEM

However, in the technique described in the above literature, paragraph [0013] describes "it is possible to intuitively grasp the progress of treatments, that is, the recovery of motor functions or reflect it on a current action as needed", and therefore, the purpose of treatments is "recovery of motor functions". For this reason, in the conventional technique, cognitive function determination is not performed.

The present invention enables to provide a technique of solving the above-described problem.

### SOLUTION TO PROBLEM

According to the invention, there is provided the subject matter of the independent claims. Some further aspects are defined in the dependent claims. The embodiments that do not fall under the scope of the claims are to be interpreted as examples useful for understanding the disclosure.

### ADVANTAGEOUS EFFECTS OF INVENTION

According to the present invention, it is possible to accurately evaluate the cognitive ability of a user.

### BRIEF DESCRIPTION OF DRAWINGS

Fig. 1 is a block diagram showing the configuration of a cognitive ability estimation apparatus according to the first example embodiment;
Fig. 2 is a block diagram showing the configuration of a rehabilitation support system according to the second example embodiment;
Fig. 3 is a view showing an example of the operation panel screen of the rehabilitation support system according to the second example embodiment;
Fig. 4 is a view showing an example of the task data table of the rehabilitation support system according to the second example embodiment;
Fig. 5 is a view showing an example of a display screen on the head mounted display of the rehabilitation support system according to the second example embodiment;
Fig. 6 is a view showing an example of a display screen on the head mounted display of the rehabilitation support system according to the second example embodiment;
Fig. 7 is a view showing an example of a display screen on the head mounted display of the rehabilitation support system according to the second example embodiment;
Fig. 8 is a view showing an example of a display screen on the head mounted display of the rehabilitation support system according to the second example embodiment;
Fig. 9 is a flowchart showing the procedure of cognitive ability estimation processing of the rehabilitation support system according to the second example embodiment; and
Fig. 10 is a view showing an example of a table for driving risk evaluation and falling risk evaluation based on a cognitive ability level estimated by the rehabilitation support system according to the second example embodiment.

### DESCRIPTION OF EXAMPLE EMBODIMENTS

Example embodiments will now be described in detail with reference to the drawings. It should be noted that the relative arrangement of the components, the numerical expressions and numerical values set forth in these example embodiments do not limit the scope of the present invention.

### [Related Art Technique]

There are a lot of indices for evaluating a cognitive level, including MMSE (Mini-Mental State Examination), HDS-R (Hasegawa's Dementia Scale-Revised), MoCA (Montreal Cognitive Assessment), TMT (Trail-Making Test), and FAB (Frontal Assessment Battery).

However, these indices require questions or tasks on paper to a user and therefore are evaluation indices requiring language elements or including many elements such as literacy and writing ability, which are irrelevant to pure cognition.

Aiming at correctness, a time of 15 to 20 min or more is needed for examinations. If examinations are repeated in a short period, the user learns answers to questions. Alternatively, if the condition is mild, there exists a so-called ceiling effect and, for example, the cognitive function is hardly reflected on the examination result. As described above, these methods can be used only to evaluate the cognitive level in a specific range.

Hence, there is a demand for a cognitive ability evaluation method that does not depend on literacy, writing ability, or a language, can be performed in a short period, can estimate various cognitive ability levels, and can avoid a situation where a subject knows answers due to repetitive examinations.

If such a method is established, training according to the estimated cognitive function can continuously be conducted, and this is expected to be effective for establishment of function improvement of cognitive ability (https://www.neurology-jp.org/guidelinem/degl/degl_2017_02.pdf, p. 26).

### [First Example Embodiment]

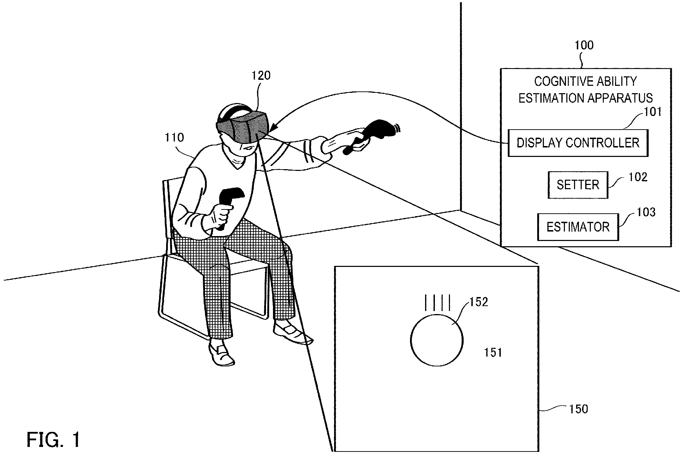

A cognitive ability estimation apparatus 100 according to the first example embodiment will be described with reference to Fig. 1. The cognitive ability estimation apparatus 100 is an apparatus for estimating the cognitive ability level of a user.

As shown in Fig. 1, the cognitive ability estimation apparatus 100 includes a display controller 101, a setter 102, and an estimator 103.

The display controller 101 generates and displays, in a virtual space 150, a target object 152 configured to urge a user 110 to do a three-dimensional body action.

As the attributes of the target object 152 in the virtual space 150, the setter 102 can set at least one of the moving speed, the number of displayed objects, the size, the display position, and the display interval.

If the user 110 achieves the body action to the target object 152, the estimator 103 estimates the cognitive ability level of the user 110 in accordance with the attributes of the target object 152. As for the estimation of the cognitive ability level, one body action is defined as one task, and the estimation may be performed based on one task, or may be done based on the results of a plurality of tasks.

With the above-described configuration, it is possible to estimate the cognitive ability level of the user more accurately.

### [Second Example Embodiment]

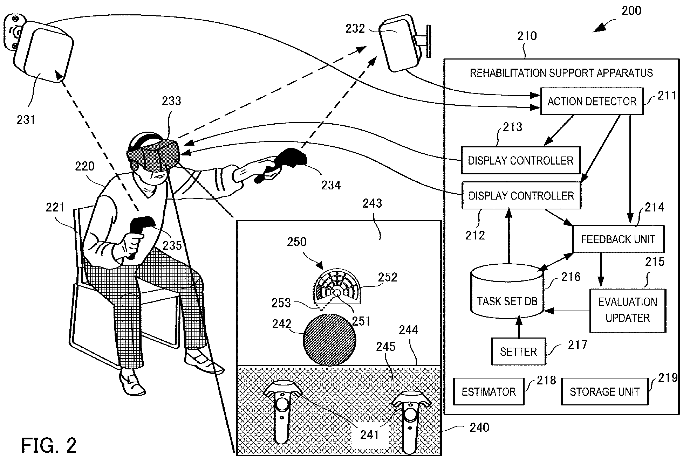

A rehabilitation support system 200 according to the second example embodiment will be described with reference to Fig. 2 Fig. 2 is a view for explaining the configuration of the rehabilitation support system 200 according to this example embodiment.

As shown in Fig. 2, the rehabilitation support system 200 includes a rehabilitation support apparatus 210, two base stations 231 and 232, a head mounted display 233, and two controllers 234 and 235. A user 220 sitting on a chair 221 twists the upper half body or stretches the hands in accordance with display on the head mounted display 233, thereby making a rehabilitation action. In this example embodiment, a description will be made assuming rehabilitation performed while sitting on a chair. However, the present invention is not limited to this. The rehabilitation action may be made while standing or walking, or on a bed, at a supine position, or at a prone position. Alternatively, the rehabilitation action may be made while running or making another specific action or motion. In addition, the controllers may be held on or attached to body parts other than hands, such as feet or trunk.

The two base stations 231 and 232 sense the motion of the head mounted display 233 and the motions of the controllers 234 and 235, and send these to the rehabilitation support apparatus 210. The rehabilitation support apparatus 210 performs display control of the head mounted display 233 based on the motion of the head mounted display 233. The rehabilitation support apparatus 210 also evaluates the rehabilitation action of the user 220 based on the motions of the controllers 234 and 235. Note that the head mounted display 233 can be of a non-transmissive type, a video see-through type, an optical see-through type, or a spectacle type. In this example embodiment, a virtual space of VR (Virtual Reality) is presented to the user. However, a physical space and a virtual space may be displayed in a superimposed manner, like AR (Augmented Reality), physical information may be reflected on a virtual space, like MR (Mixed Reality), or a hologram technology may be used as an alternative means.

In this example embodiment, as an example of a sensor configured to detect the position or action of the hand or head of the user, the controllers 234 and 235 held in the hands of the user 220, and the base stations 231 and 232 have been described. However, the present invention is not limited to this. A camera (including a depth sensor) configured to detect the positions or action of the hands themselves of the user by image recognition processing, or a sensor configured to detect the positions of the hands of the user by a temperature may be used. A wristwatch-type wearable terminal put on an arm of the user, a motion capture device, or the like can also be applied to the present invention interlockingly with an action detector 211. That is, using a three-dimensional tracking device such as Kinect^{®} or an action analysis device or attaching a marker to the body is also one example embodiment.

The rehabilitation support apparatus 210 includes the action detector 211, display controllers 212 and 213, a feedback unit 214, an evaluation updater 215, a task set database 216, a setter 217, an estimator 218, and a storage unit 219.

The action detector 211 acquires, via the base stations 231 and 232, the positions of the controllers 234 and 235 held in the hands of the user 220, and detects the rehabilitation action of the user 220 based on changes in the positions of the hands of the user 220.

The display controller 212 generates and displays, in a virtual space 240, a target object 242 configured to urge the user 220 to do a three-dimensional body action. In particular, the display controller 212 generates, in the virtual space, avatar objects 241 that move in accordance with a detected rehabilitation action and the target object 242 representing the target of the rehabilitation action. The display controller 212 displays, on a display screen (in the virtual space 240), the images of the avatar objects 241 and the target object 242 in accordance with the direction and position of the head mounted display 233 detected by the action detector 211. The images of the avatar objects 241 and the target object 242 are superimposed on a background image 243. Here, the avatar objects 241 have the same shape as the controllers 234 and 235. However, the present invention is not limited to this, and the size, shape, or color may be changed on the left and right sides. The avatar objects 241 move in the virtual space 240 in accordance with the motions of the controllers 234 and 235. The controllers 234 and 235 are each provided with at least one button and configured to perform various kinds of settings including initial settings such as origin setting by operating the button. The button can be disabled, or instead of arranging the button, all settings may be executed separately using an external operation unit. The background image 243 is cut out from a virtual space including a horizontal line 244 and a ground surface image 245.

The display controller 212 generates the target object 242 in the virtual space and moves this downward from above the user 220. Accordingly, on the head mounted display 233, the target object is displayed such that the display position and size gradually change (for example, gradually becomes large and then becomes small). The user 220 moves the controllers 234 and 235 to make the avatar objects 241 in the screen close to the target object 242. Note that as for the moving direction of the target object, for example, the target object may be displayed such that it rises from the floor surface to above the head. Also, including movement in the depth direction or the left-and-right direction in addition to the movement in the vertical direction, any three-dimensional movement may occur, or the target object may be fixed at a specific coordinate position without moving.

The user 220 moves the controllers 234 and 235 to bring the avatar objects 241 in the screen close to the target object 242. If the avatar object 241 hits the target object 242, the display controller 212 causes the target object 242 to disappear, and the feedback unit 214 determines that the target action is achieved, and displays a message. More specifically, if the shortest distance between the target object 242 and a sensor object included in the avatar object 241 falls within a predetermined range, the target is achieved, and the target object 242 disappears. At this time, if the shortest distance between the target object 242 and the sensor object (for example, a spherical object including the center point of the avatar object 241) included in the avatar object 241 is equal to or less than a predetermined threshold S1, the target is completely achieved. In a case of complete achievement, "excellent" is displayed, and a corresponding voice is output simultaneously to make feedback. At the same time, the controller 234 or 235 held by the hand that has achieved the task may be vibrated, or stimuli may be imparted to the sense of smell or the sense of taste. The rehabilitation action may be evaluated in three or more levels depending on how much the distance between the sensor object and the target object 242 decreases. Also, two or more target objects may be generated simultaneously in the virtual space and displayed. Notifications of task achievement for five sense stimulation may be combined in any way in accordance with the action type or the like. If the shortest distance between the target object 242 and the sensor object included in the avatar object 241 is not less than the threshold S1 and not more than a threshold S2, "well done" is displayed because of the achievement of the target, and a corresponding voice is output to make feedback. Note that the output voice need not be the same as the displayed message, and a nonverbal sound effect, for example, "dingdong" may be used.

The display controller 213 displays a radar screen image 250 on the display screen of the head mounted display 233. The radar screen image 250 is a notification image used to notify the user of generation of the target object 242. The radar screen image 250 notifies the user of the direction in which the generated target object 242 is located relatively with respect to a reference direction (initially set in the front direction of the user who is sitting straight on a chair) in the virtual space. The radar screen image 250 also notifies the user how far the position of the generated target object 242 is apart from the user 220. Note that the notification image is not limited to the radar screen image, and the notification may be made using characters, an arrow, a symbol, an illustration, or a type, intensity, blinking, or the like of light or a color. The notification method is not limited to the image, and may use a voice, a vibration, or a combination of some of a voice, a vibration, and an image. Independently of the direction of the head of the user 220, the display controller 213 displays the radar screen image 250 at the center (for example, within the range of -50° to 50°) of the display screen of the head mounted display 233. However, the display portion is not limited to the center, and may be, for example, an arbitrary place on the four corners, the upper end, the lower end, the left end, and the right end of the screen.

The radar screen image 250 includes a head image 251 representing the head of the user viewed from above, a block image 252 obtained by dividing the periphery of the head image 251 into a plurality of blocks, and a fan-shaped image 253 as a visual field image representing the visual field of the user. A target position image representing the position of a target object is shown by coloring, blinking, or lighting a block in the block image 252. This allows the user 220 to know whether the target object exists on the left side or the right side with respect to the direction in which he/she faces. Note that in this example embodiment, the block image 252 is fixed, and the fan-shaped image 253 moves. However, the present invention is not limited to this, and the block image 252 may be moved in accordance with the direction of the head while fixing the fan-shaped image 253 and the head image 251. More specifically, if the head turns to the left, the block image 252 may rotate to right.

The feedback unit 214 preferably changes the message type via the display controller 212 in accordance with evaluation of the rehabilitation action. For example, if the sensor object contacts the center of the target object 242, "excellent" is displayed. If the sensor object contacts only the peripheral portion of the center of the target object 242, "well done" is displayed. The size of the target object 242 and the size of the peripheral portion can be set by the setter 217. The size of the sensor object can also be set by the setter 217.

The feedback unit 214 performs feedback to impart stimuli to two or more of the five senses (sense of sight, sense of hearing, sense of touch, sense of taste, and sense of smell) of the user who has virtually touched the target object 242. The feedback is performed almost at the same time as the timing at which the sensor object enters a predetermined distance from the center of the target object 242 or the timing at which the sensor object contacts the target object 242 (real-time multi-channel biofeedback). The effect is large if a delay from the timing to feedback is, for example, 1 sec or less. The shorter the interval between the operation timing of the user and the timing of feedback is (the smaller the delay is), the larger the effect is. While performing feedback of giving stimuli to the sense of sight of the user by an image "excellent! ", the feedback unit 214 simultaneously performs feedback of giving stimuli to the sense of hearing of the user by a voice output from a speaker 236. The stimulating feedback to the five senses (sense of sight, sense of hearing, sense of touch, sense of taste, and sense of smell) is performed to notify the user of the presence/absence of achievement in each task.

Also, the feedback unit 214 may simultaneously output feedback of giving stimuli to the sense of sight of the user 220 by the image "excellent!", feedback of giving stimuli to the sense of hearing of the user 220 by the voice output from the speaker, and feedback of giving stimuli to the sense of touch of the user 220 by causing the controller 234 to vibrate. Alternatively, the feedback unit 214 may simultaneously output only two types of feedback, including feedback of giving stimuli to the sense of sight of the user 220 by the image "excellent! ", and feedback of giving stimuli to the sense of touch of the user 220 by causing the controller 234 to vibrate. The feedback unit 214 may simultaneously output only two types of feedback, including feedback of giving stimuli to the sense of hearing of the user 220 by a voice "excellent!", and feedback of giving stimuli to the sense of touch of the user 220 by causing the controller 234 to vibrate.

The action of the user 220 moving the controllers 234 and 235 is a rehabilitation action, and display of a target object for urging the user 220 to do one rehabilitation action that he/she should perform is called a task. Information (task data) representing one task includes at least the moving speed, the number of displayed objects, the size, the display position, and the display interval as the attributes of the target object 242 in the virtual space 240. The task data may also include the appearance direction of the target object (right 90°, right 45°, front, left 45°, and left 90° with respect to the front direction of the chair), the distance to the target object, the shape (size) of the target object, the appearance position (the depth direction distance from the user), the appearance interval (time interval), the moving speed in falling, rising, or the like, the color of the target object, which one of the left and right controllers should be used to acquire, the number of target objects that simultaneously appear, the size of the sensor object, and the like. The distance from the user 220 to the fall position of the target object 242 in the depth direction may continuously be set, or may be set to one of, for example, three stages. For example, a change can be made such that the target object falls quite near the user 220 or falls to a position that the user 220 cannot reach unless largely inclining the body forward. This can control an exercising load to be given to the user, and a load to a spatial cognitive ability or a spatial comprehension ability.

The evaluation updater 215 evaluates the rehabilitation action of the user in accordance with the amount and quality of the task achieved by the user 220 and adds a point. Here, the quality of the achieved task includes "well done" or "excellent", that is, how close the avatar object could be brought to the target object. The evaluation updater 215 adds different points to achieved tasks (a high point to a far object, and a low point to a close object). The evaluation updater 215 can update a task in accordance with the integrated point. For example, a task (the attribute of a target object) may be updated using a task achievement ratio (the number of achieved targets/the number of tasks). The evaluation updater 215 compares the rehabilitation action detected by the action detector 211 and a target position represented by the target object displayed by the display controller 212, and evaluates the rehabilitation capability of the user 220. More specifically, it is decided, by comparing the positions in the three-dimensional virtual space, whether the target object 242 and the avatar object 241 that moves in correspondence with the rehabilitation action detected by the action detector 211 overlap. If these overlap, it is evaluated that one rehabilitation action is cleared, and a point is added. The display controller 212 can make the target object 242 appear at different positions (for example, positions of three stages) in the depth direction. The evaluation updater 215 gives different points (a high point to a far object, and a low point to a close object).

The evaluation updater 215 updates a target task in accordance with the integrated point. For example, a target task may be updated using a task achievement ratio (the number of achieved targets/the number of tasks).

The task set database 216 stores a set of a plurality of tasks. A task represents one rehabilitation action that the user should perform. More specifically, as information representing one task, the position of a target object that appears, its speed and size, and the size of an avatar object at that time, and the like are stored. The task set database 216 stores a task set that decides the order of providing the plurality of tasks to the user. For example, task sets may be stored as templates for each hospital, or a history of executed task sets may be stored for each user. The rehabilitation support apparatus 210 may be configured to be communicable with another rehabilitation support apparatus via the Internet. In this case, one task set may be executed by the same user in a plurality of places, or various templates may be shared by a plurality of users in remote sites.

As the attributes of the target object 242 in the virtual space 240, the setter 217 sets at least one of the moving speed, the number of displayed objects, the size, the display position, and the display interval. If the user 220 can achieve a body action to the target object 242, the estimator 218 estimates the cognitive ability level of the user 220 in accordance with the attributes of the target object 242. The setter 217 can set a delay time from the timing of notifying the generation of the target object 242 to the timing of generating the target object 242, thereby controlling a cognitive load given to the user 220. That is, the user needs to continuously memorize and hold an action that he/she should perform during the time after he/she knows, by the radar screen image 250 or the like, a position in the virtual space where the target object is generated (a position representing in which direction the head mounted display should directed to display the target object) until actual generation of the target object. The "memorization time" is a cognitive load for the user. Also, the setter 217 may control the cognitive load by changing the time not "until the timing of generating the target object 152" but "until the target object 152 approaches the range the user 220 can reach". The setter 217 may give a cognitive load to the user 220 by displaying the background image 243 other than the target object 242 on the head mounted display 233.

Note that when changing the cognitive load, it is preferable to notify the user in advance that the cognitive load is to be increased or decreased. As for the notification method, the notification may be done by visually using characters or a symbol, by a voice, or by touching a part of the body, for example, by tapping a shoulder, an elbow, an arm, or a foot.

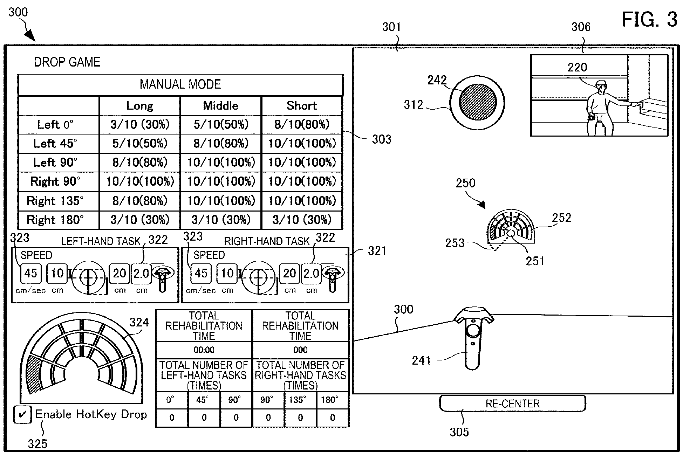

Fig. 3 is a view showing an example of a screen (operation panel) 300 to be operated by an operator. The setter 217 displays the operation panel 300. In this example embodiment, seven parameters (distance, height, angle, size, speed, sensitivity, and interval) are set by the intuitive operation panel 300, thereby generally evaluating a posture balance ability and a dual task type cognitive processing ability. Measurement can be done by one of a manual mode (a method of setting parameters of each task and performing an operation on a task basis), a template mode (a method of setting parameters for a plurality of task sets in advance), and an auto mode in which a device is caused to automatically generate a task, or a combination thereof. Note that it is also possible to, on the operation panel, confirm the basic information of the user and various kinds of cognitive and motor functions evaluation indices and examination results, create a template, and set and instruct the auto mode.

The display that displays the operation panel 300 can be any device, and may be an external display connected to the rehabilitation support apparatus 210 or a display incorporated in the rehabilitation support apparatus 210. The operation panel 300 includes a user visual field display region 301, various parameter setting regions 321 to 324, a score display region 303, and a re-center button 305. For a descriptive convenience, Fig. 3 includes a region 306 showing the actual state of the user 220. However, the operation panel 300 need not include the region 306.

The user visual field region 301 shows an image actually displayed on the head mounted display 233. A reference direction in the virtual space is displayed in the user visual field region 301. As described with reference to Fig. 2, the radar screen image 250 is displayed at the center (for example, within the viewing angle range of -50° to 50°) of the user visual field region 301. The radar screen image 250 shows the relative direction of the position of the target object 242 that appears next with respect to the reference direction in the virtual space. In this example, the coloring position in the block image 252 represents that the target object 242 appears at the farthest position on the left side with respect to the reference direction 311 in the virtual space. Based on the position of the fan-shaped image 253 and the direction of the head image 251, it can be seen that the user already faces left.

The various parameter setting regions 321 to 324 are screens configured to set a plurality of parameters for defining a task. The setter 217 can accept inputs to the various parameter setting regions 321 to 324 from an input device (not shown). The input device may be a mouse, a ten-key pad, or a keyboard, or may be various kinds of controllers, a joystick for game, or a touch panel, and can use any technical component.

The various parameter setting regions 321 to 324 include the input region 321 that decides the sizes of left and right target objects, the input region 322 that decides the size of the sensitivity range of the avatar object 241, the input region 323 that decides the moving speed of the target object, and the input region 324 that decides the position of a target object that appears next. The operation panel 300 also includes a check box 325 that sets whether to accept an operation of a target object appearance position by an input device (hot key).

The input region 321 can set, on each of the right and left sides, the radius (visual recognition size) of a visual recognition object that makes the target object position easy for the user to see, and the radius (evaluation size) of a target object that reacts with the avatar object 241. That is, in the example shown in Fig. 3, the user can see a circle with a radius of 20 cm. Actually, the task is correctly completed only when he/she has touched a ball with a radius of 10 cm located at the center of the circle. If the visual recognition size is small, it is difficult for the user to find the target object. If the visual recognition size is large, the user can easily find the target object. If the evaluation size is large, the allowable amount of the deviation of the avatar object 241 is large. If the evaluation size is small, the allowable amount of the deviation of the avatar object 241 is small, and a rehabilitation action can be evaluated more severely. The visual recognition sizes and the evaluation sizes may be made to match.

In the input region 322, the left and right sensor sizes of the avatar object 241 (the size of the sensor range of the sensor object) can separately be set. If the sensor size is large, a task is achieved even if the position of a hand largely deviates from the target object. Hence, the difficulty of the rehabilitation action is low. Conversely, if the sensor size is small, it is necessary to correctly move the hand to the center region (evaluation size) of the target object. Hence, the difficulty of the rehabilitation action is high. In the example shown in Fig. 3, the sensor sizes are 2 cm on the left and right sides.

In the input region 323, the speed of the target object 242 moving in the virtual space can be defined on each of the left and right sides. In this example, the speed is set to 45 cm/s.

The input region 324 is an image used to input the position of the next position (the distance to the task and the angle), and has the shape of the enlarged radar screen image 250. Since the check box 325 has a check mark, if an operation of clicking or tapping one of a plurality of blocks in the input region 324 is performed, the target object 242 is generated at a position in the virtual space corresponding to the position of the block for which the operation is performed.

That is, in the example shown in Fig. 3, the task for the user 220 is to bring an avatar object (controller) including a sensor portion with a size of 2 cm into contact with a target object (ball) having a radius of 10 cm that falls at a speed of 45 cm/s in a far place on the left side in a good timing in the virtual space. Fig. 3 shows a state in which the target object 242 appears in the user visual field region 301. In this state, when the user 220 stretches the left arm, as shown in the region 306, the avatar object 241 appears in the user visual field region 301. A visual recognition object 312 that raises the visibility of the target object 242 is displayed around the target object 242. The visual recognition size set in the input region 321 is the radius of the visual recognition object 312 with a doughnut shape.

At the point of time when the avatar object 241 contacts the visual recognition object 312, the target object 242 does not disappear, and correct achievement of the task is not obtained (a predetermined point is added, and good evaluation is done). Correct achievement of the task (perfect evaluation) is obtained only when the avatar object 241 contacts the target object 242.

On the other hand, the total number of tasks at each position, and a count point representing how many times a task has been achieved are shown in the score display region 303. The point may be expressed as a fraction or a percentage, or a combination thereof. After a series of rehabilitation actions decided by one task set, the feedback unit 214 derives a rehabilitation evaluation point using values in the score display region 303.

An example of calculation of the rehabilitation evaluation point is as follows. Rehabilitation evaluation point = 100 × ([determination score × count of Short] + [determination score × count of Middle × 1.1] + [determination score × count of Long × 1.2] + [count of five continuous Perfect catch × 10]), determination score: Perfect (excellent) = 2, Good (well done) = 1, Failure = 0

The re-center button 305 is a button that accepts, from the operator, a reconstruction instruction for reconstructing the virtual space in accordance with the position of the user 220. If the re-center button 305 is operated, the display controller 212 sets the position of the head mounted display 233 at the instant to the origin, and reconstructs the virtual space having, as the reference direction, the direction of the head mounted display 233 at that instant.

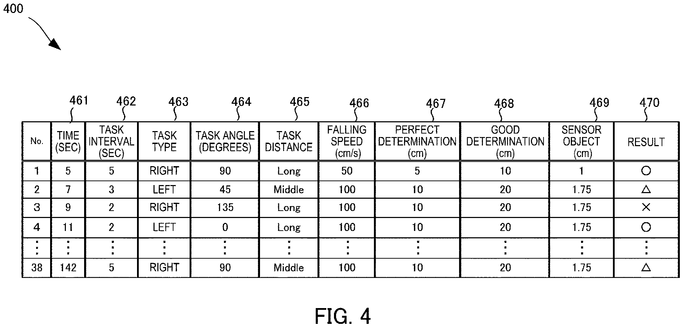

Fig. 4 is a view showing a task table 400 stored in the task set database 216. In the task table 400, a time (task generation timing) 461, a task interval 462 from the end of an immediately preceding task, a task type 463, a task angle 464, and a task distance (intensity) 465, and a task distance (intensity) 465 are stored in linkage with a task ID. Also, in the task table 400, a target object speed 466, a perfect determination (excellent evaluation) reference size 467, a good determination (well done evaluation) reference size 468, a sensor object size 469, a task achievement result, and the like are stored in linkage with a task ID. In addition to these, a delay time (predetermined time) from task generation notification to task generation may be set for each task.

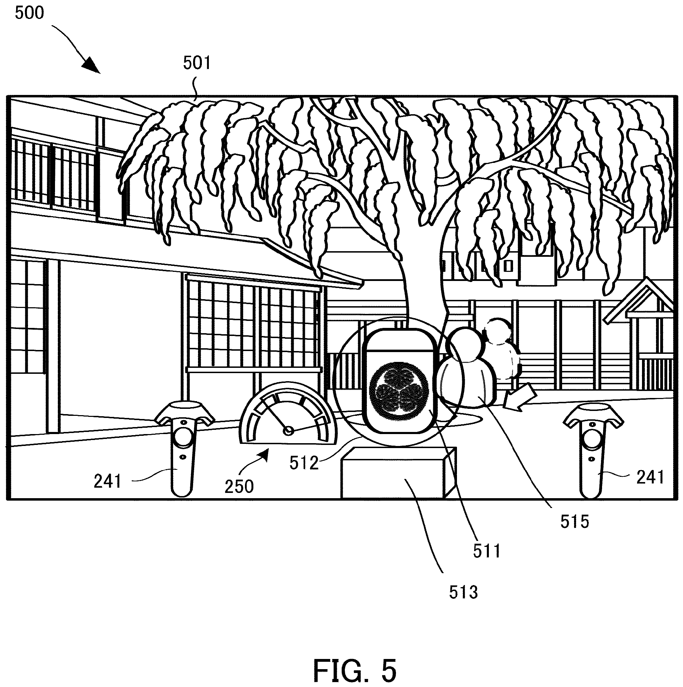

Figs. 5 to 7 are views showing examples of display on the head mounted display 233 according to this example embodiment. In Fig. 5, an image showing an inro 511 as a target object is displayed on a background image 501 expressing a street in the Edo period. In addition, under the inro 511, a sen-ryo-bako 513 is displayed as an item to be protected by the user, and a ninja 515 gradually comes close from the far side. The speed of the ninja 515 is the speed set in the input region 323 of the operation panel 300 (the speed here has the same meaning as a time limit). A circle 512 serving as a visual recognition assisting image is displayed on the inro 511. If the user touches the inro 511 by the sensor object (the center of the tip of the avatar object 241) before the ninja 515 reaches the sen-ryo-bako 513, the task is achieved. Two types of circles, that is, red and blue circles are prepared as the circle 512. The task for the inro 511 surrounded by the red circle 512 is to operate the red avatar object 241 on the right side, which corresponds to the controller 235 held in the right hand, and bring it into contact with the inro 511. On the other hand, the task for the inro 511 surrounded by the blue circle 512 is to operate the blue avatar object 241 on the left side, which corresponds to the controller 234 held in the left hand, and bring it into contact with the inro 511.

The inro 511 is displayed at a position (depth and angle) set in the input region 324 of the operation panel 300. The position of the inro 511 does not change until the user makes the avatar object 241 touch it in the virtual space. That is, the inro 511 is a target object fixed in the space (also called a horizontal task because the user is required to horizontally stretch the body). Such a fixed target object is very effective as rehabilitation for a disease such as cerebellar ataxia. That is, an image of a limited body motion can be imprinted, by feed forward, in the brain of a patient who has forgotten how to move his/her body. If the distance of the inro 511 in the depth direction is increased, the motion intensity can be changed. Also, if multi-channel biofeedback by five sense stimulation for notifying the achievement of each task is combined, the memory can easily be fixed in the brain, and exercise ability greatly improves. Furthermore, according to such a horizontal task, chronic pains can be improved along with the reconstruction of cerebral cortex. Alternatively, it is possible to recover cognitive impairment called chemobrain or a phenomenon that the position sense of a cancer patient using an anticancer drug lowers due to neuropathy. The place where the target object appears may be notified in advance to give a hint and reduce the cognitive load. Touch notices by touching the body without any language is effective to reduce the cognitive load. a plurality of repeated language notices are also effective to reduce the cognitive load. As for the method of language informing, the cognitive load may be reduced by giving a simple short instruction close to the imperative form. Alternatively, a more complex instruction may be given in a questioning format, for example, using "blue? (take by the right hand)". Language informing may be given in a form including a cognitive task such as calculation, for example, "take by the right hand if you hear a number divisible by 2". Note that not only the horizontal position and depth where the inro 511 is generated but also a height may be set.

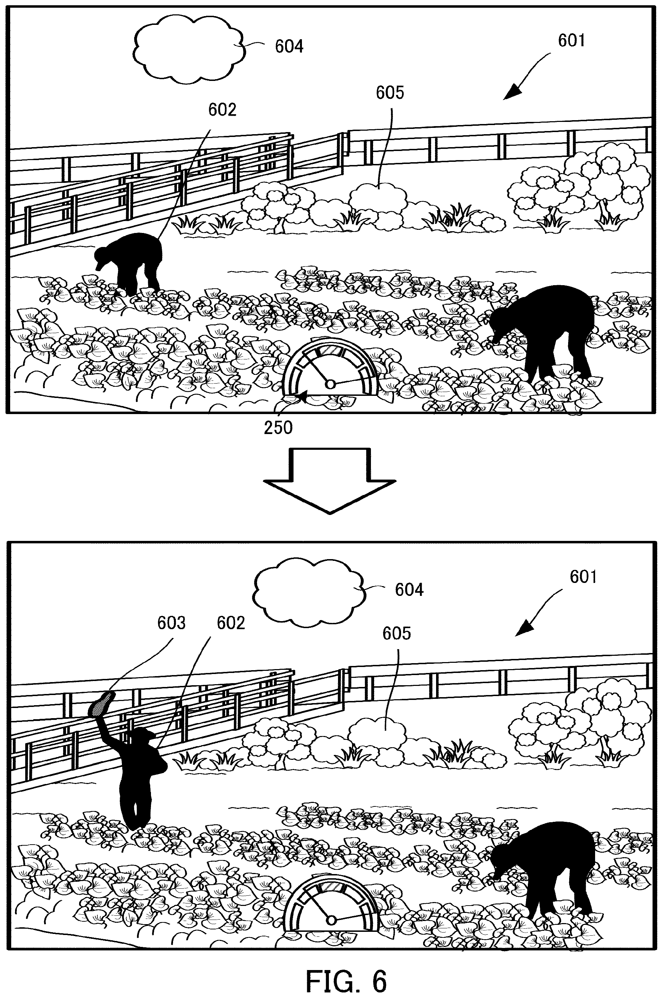

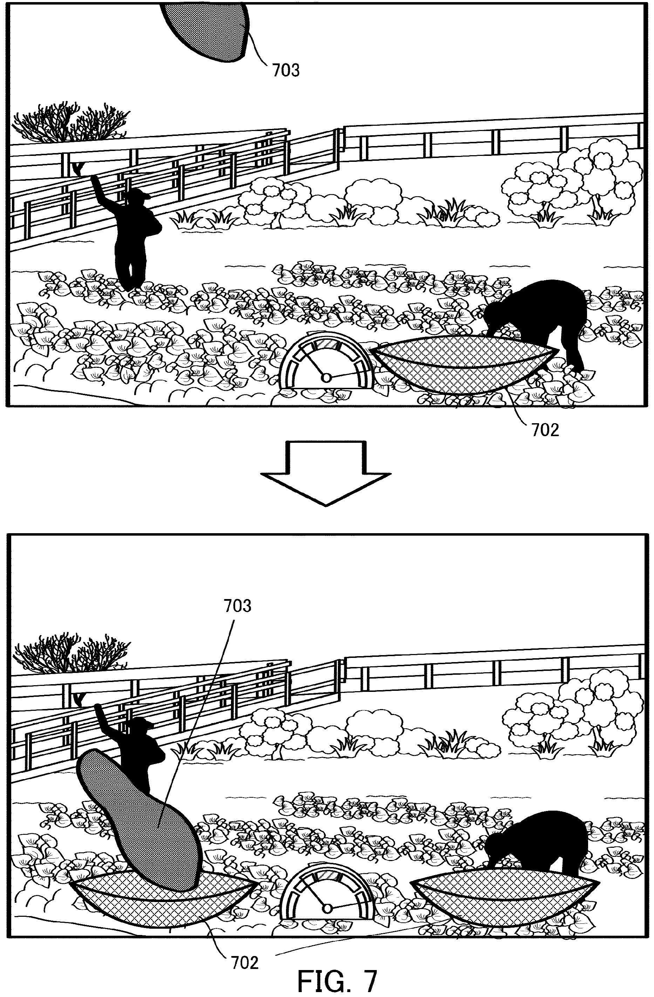

Fig. 6 is a view showing an example (vertical task) of a screen for performing a task in which a target object moves vertically. In Fig. 6, on a background image 601 representing a field, an image of a person representing a farmer is displayed as a trigger object 602 serving as a trigger of target object appearance. That is, the display controller 213 displays the trigger object 602 as a notification image used to notify the user of generation of a target object 603. When a predetermined time elapses after the trigger object 602 throws up the target object 603 in the shape of a potato, a target object 703 having the shape of a large potato appears from the upper side of the screen, as shown in Fig. 7. When the falling target object 703 is received by moving an avatar object 702 having the shape of a basket, the task is achieved. The left and right avatar objects 702 move on the screen in synchronism with the motions of the controllers 234 and 235.

The setter 217 can set the delay time from the timing at which the trigger object 602 throws up the target object 603 and notifies the generation of the target object 603 until generation of the target object 703. Thus, the cognitive load given to the user can be adjusted. Note that in synchronism with the motion of the trigger object 602, generation of the target object may be notified at the same timing using the radar chart type notification image 250, or a notification by a voice may be combined.

In this way, the setter 217 can give a cognitive load to the user not only by a task of a background including only a horizontal line as shown in Fig. 2 but by a task of a background including a large quantity of information as shown in Figs. 5 and 6. That is, it is made difficult to memorize that the target object 603 has appeared, and the position to which the target object 703 is expected to fall, thereby giving a load closer to a cognitive load necessary in a real life to the user of rehabilitation.

In particular, the setter 217 changes the mode of the task and changes at least a part of the background image 301 along with time, thereby giving a cognitive load to the user 220. In the example shown in Fig. 6, for example, in the background image 601, a cloud 604 may be moved, plants 605 may be shaken, or an animal (not shown) irrelevant to the target object may be made to appear. This can impede concentration to the target object 603 and make it more difficult for the user 220 to memorize the position to which the target object 603 is expected to fall. More technically, it can be said that information irrelevant to the task is displayed on the background image to prepare an environment in which it is difficult to concentrate to the target object and intentionally cause an attention disorder (more specifically, a selective attention disorder, a divided attention disorder, a conversion attention disorder, or a sustained attention disorder), thereby making memorization difficult and controlling the cognitive load.

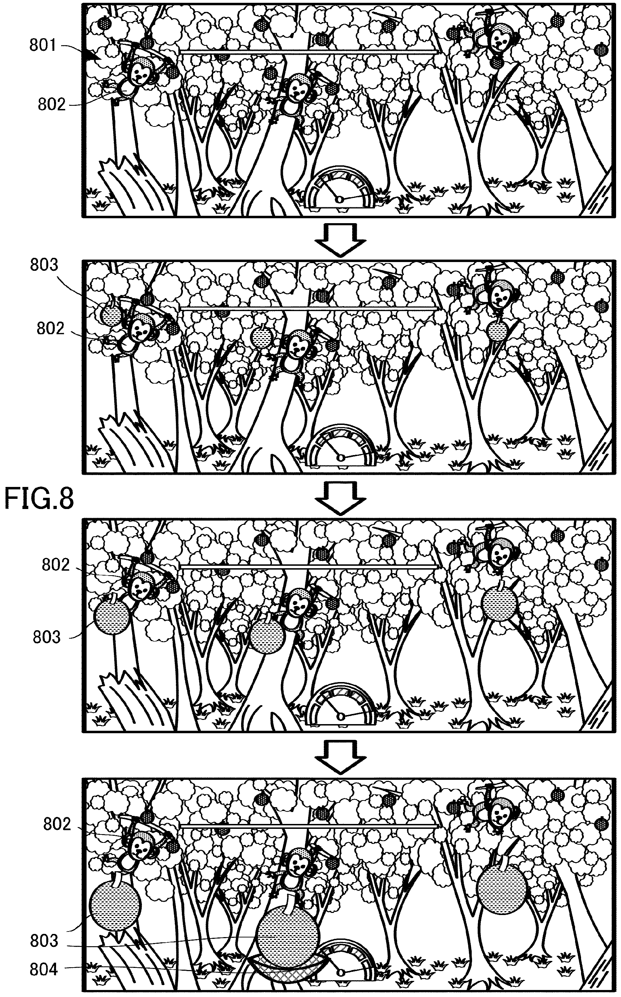

Fig. 8 is a view showing another example (vertical task) of display on the display screen according to this example embodiment. In Fig. 8, in a background image 801 like woods, a trigger object 802 representing a monkey and a target object 803 representing an apple are displayed. When the trigger object 802 representing a monkey drops the target object 803 representing an apple from a tree, and the target object 803 approaching the user is received by moving an avatar object 804 representing a basket, the task is achieved. In this case as well, the setter 217 starts dropping the target object 803 after the elapse of a predetermined time from the timing at which the trigger object 802 shakes the tree and notifies the generation of the target object 803, thereby giving a cognitive load to the user 220 while causing an attention disorder.

Also, the setter 217 causes at least two to five target objects 803 to simultaneously exist in the three-dimensional virtual space 240 and displays these on the display screen, thereby giving a cognitively stronger load to the user 220. In other words, the setter 217 generates the at least two target objects 803 at different positions in the left-and-right direction in the three-dimensional virtual space.

In particular, if the at least two target objects 803 are generated at a plurality of positions in a direction (the left-and-right direction in Fig. 8) different from the moving direction (the falling direction in Fig. 5) of the target object 803, a larger cognitive load can be given. That is, since the user 220 needs to move the controllers 234 and 235 in consideration of the movement in the vertical direction, the difference between the generation positions in the left-and-right direction, and the difference in the falling position in the depth direction, the spatial cognitive ability is also tested. As described above, the type, number, size, spatial spread, position, amount, and the like of information included in a notification image including a trigger object or a notification sound are adjusted in addition to the change of the predetermined time of the task. It is therefore possible to quantitatively adjust and control the complexity of information to be memorized and held, that is, a cognitive load that should be subjected to information processing by the brain of the user.

The evaluation updater 215 evaluates the cognitive ability of the user using information such as whether the avatar object has reached, in a good timing, a three-dimensional target position represented by the target object, the time interval from target object generation notification to generation and the number of target objects, and the degree of the load that causes the attention disorder on the background image.

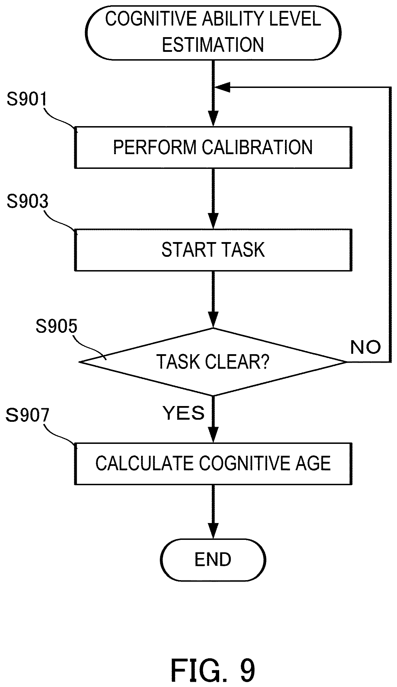

Fig. 9 is a flowchart showing the procedure of cognitive ability estimation processing of the rehabilitation support apparatus 210. In step S901, as calibration processing, the target of a rehabilitation action is initialized in accordance with the user. More specifically, each user is first made to do a work for acquiring an action enable range as calibration. The range is set to the initial value, and the target is initialized in accordance with the user. Also, more specifically, the operator reads out a task set (a horizontal task set or a vertical task set) formed by a plurality of tasks from the task set database. Here, as an example, size of the target object is 10 cm, the display interval of the target object is 1 sec, target objects generated as one task include one to five objects, and the generation ranges are set to three patterns, that is, 90°, 45° to 135°, and 0° to 180°. Three task sets are generated at an interval of 4 sec.

Next, in step S903, a task (that is, display of the target object and evaluation of achievement by an avatar object by the display controller 212) is started.

In step S905, if all the tasks in the three continuous task sets obtain perfect determination, it is determined that the task sets are achieved, and the process advances to step S907. In accordance with the attribute of the target object in the task sets, the estimator 218 calculates the cognitive ability as, for example, a cognitive age. The attribute parameters used by the estimator 218 can variously be selected. The cognitive ability level of the user is calculated based on at least the moving speed of the target object and the number of displayed target objects (for example, one to five) in one task set. The estimator 218 calculates the cognitive ability level using different parameters or different parameter coefficients between a case where the target object moves in the vertical direction (vertical task) and a case where the target object does not move in the vertical direction (horizontal task). The estimator 218 calculates the cognitive ability level of the user using different parameters or different parameter coefficients in accordance with the background of the screen on which the target object is displayed. That is, the cognitive ability level is evaluated high for the user who has achieved the task on a complex background. Note that for an adult, a high cognitive ability level and a young cognitive age can be considered as equivalent. The higher the moving speed of the target object in the virtual space is in a task, the higher the cognitive ability level estimated by the estimator 218 is if the user can achieve the task. The larger the number of target objects generated in one task set is in the virtual space, the higher the cognitive ability level estimated by the estimator 218 is if the user can achieve the task. Here, the number of target objects generated in one task set indicates the number of target objects that can simultaneously exist in the virtual space. That is, if the number is two, two target objects are simultaneously displayed in the virtual space at maximum. The larger the number is, the larger the number of targets that the user needs to simultaneously recognize is, and the larger the load of parallel processing in the brain is.

The shorter the interval of the appearance of the target object in the virtual space is, the higher the cognitive ability level estimated by the estimator 218 is if the user can achieve the task. The smaller the size of the target object is, the higher the cognitive ability level estimated by the estimator 218 is if the user can achieve the task. The wider the appearance range of the target object is, the higher the cognitive ability level estimated by the estimator 218 is if the user can achieve the task. By employing such an estimation method, various problems of cognitive function evaluation indices represented by MMSE, HDS-R, MoCA, TMT, and FAB can be solved. More specifically, it is possible to estimate pure cognitive ability while excluding elements irrelevant to the cognitive ability, such as literacy and writing ability, suppress the time needed for examinations to a very short time, and eliminate learning and the ceiling effect caused by repetitive examinations.

The storage unit 219 stores an equation or table representing the relationship between the cognitive age and the attribute of the target object for which the user has achieved a body action. Using the equation or the table, the estimator 218 estimates the cognitive ability level of the user as the cognitive age. The apparatus may include an updater configured to update the equation or the table stored in the storage unit 219 based on big data that associates an actual age with a result of cognitive ability estimation. Machine learning may be performed using the actual age as supervisory data. If the data of population becomes large, the cognitive ability level estimation accuracy becomes high.

The setter 217 can also set the attributes of the target object to be displayed next in accordance with the cognitive ability level of the user estimated by the estimator 218.

The estimator 218 can calculate the cognitive age as the cognitive ability using, for example, the following expressions. (1) Estimation accuracy priority (adjusted contribution, Adjusted R-squared: 0.8974, Akaike Information Criterion AIC = 2936): parameters are introduced as many as possible to obtain the highest estimation accuracy → the accuracy is so high that learning is unnecessary Cognitive age = 80 (simple background, horizontal task, number of target objects = 1, 10 cm, 90°, speed = 25 cm/sec) + [if the complexity of background is raised, -2.5 years] + [if a vertical task is possible, -10 years] + [every time the number of target objects is increased by one, -5 years] + [every time the size of the target object in 10 cm is decreased by 1 cm, -1 year] + [every time the generation range of the target object is extended by 10°, -0.4 years] + [every time the speed in the task is increased by 10cm/s, -2.4 years] (2) Practicality priority (adjusted contribution, Adjusted R-squared: 0.7806, Akaike Information Criterion AIC = 3310): while maintaining estimation accuracy at predetermined level, the number of variable parameters to be used is decreased to two Cognitive age = 84 (simple background, horizontal task, number of target objects = 1, 10 cm, 90°, speed = 25 cm/sec) + [every time the number of target objects is increased by one, -5 years] + [every time the speed in the task is increased by 10cm/s, -2.7 years]

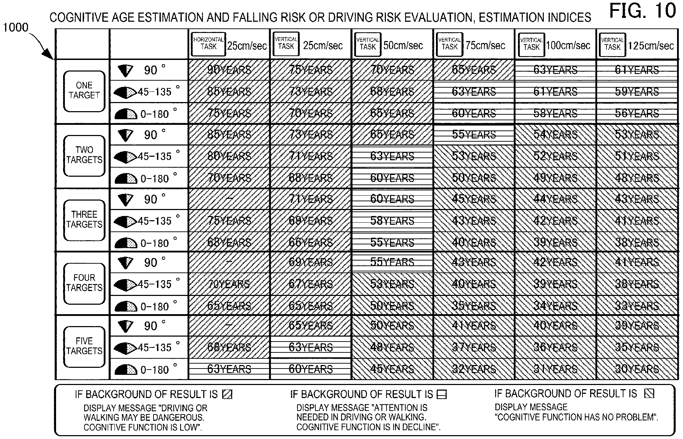

Fig. 10 shows a table 1000 in which the cognitive age calculated by the above-described equation is applied to automobile driving risk evaluation. In a case of the background as shown in Fig. 5, the age for the horizontal task in the table is decreased by 3. Similarly, in a case of the background as shown in Fig. 6, the age is decreased by 5, and in a case of the background as shown in Fig. 7, the age is decreased by 3. According to Fig. 10, a falling risk evaluation can also be performed in a similar way. Using the table shown in Fig. 10, the estimator 218 evaluates the driving risk or falling risk of the user. In addition, based on the cognitive ability level of the user estimated by the estimator 218, the setter 217 proposes, to the operator, a treatment program for improving the cognitive ability level, reducing the driving risk, or reducing the falling risk.

If the cognitive age is 65 years or more, the cognitive function is low, and it is evaluated that driving is dangerous. If the cognitive age is 55 to 65 years, the cognitive function is in decline, and it is evaluated that attention is needed in driving. On the other hand, if the cognitive age is 54 years or less, the cognitive function has no problem, and it is evaluated that the user is sufficiently capable of driving. Similarly, a falling risk in daily life may be evaluated. Here, thresholds are set to 65 years and 55 years. However, the thresholds may be changed in accordance with the required action. For example, if the user drives in a region of little traffic, it may be evaluated that driving is possible even if the cognitive age is 65 years.

Note that it is found that if the rehabilitation support system 200 according to this example embodiment is used, the cognitive function level can be improved and maintained (the effect of one training continues for three weeks, and the effect can be fixed to an almost constant level if the training is performed three times), and it is considered that for a user judged to have a risk in driving, the evaluation may be changed to "driving OK".

When estimating the cognitive age, the setter 217 may display a message concerning control of the cognitive load (attribute parameter). For example, a message "reduce the speed and test again" can be considered. A recommended numerical value may be presented by, for example, "set the speed to 25 cm/sec". Also, to correctly calculate the cognitive function level, various kinds of parameters may be controlled automatically.

If the rehabilitation support system 200 according to this example embodiment is used, it is possible to recognize the type of the lowered cognitive function and effectively conduct rehabilitation more correctly. For example, a user evaluated to be 65 years old in a vertical task at 25 cm/sec can do spatial recognition. If the speed is low (25 cm/sec), he/she can handle a multitask (four targets). For such a user, training to gradually increase the speed is effective.

As described above, in this example embodiment, the cognitive function is estimated by adjusting two or more of parameters including the time limit (falling speed or the like), the size of the target, the motion of the target, the number of targets, the display position of the target in the three-dimensional space, and background bb information for intentionally causing an attention disorder. The system according to this example embodiment is a system that needs color identification. Also, the system according to this example embodiment is a system configured to judge the type of attention disorder or treat the attention disorder. Also, the system according to this example embodiment is a system configured to evaluate or treat a falling risk or a driving risk.

According to this example embodiment, it is also possible to judge which one of a sustained attention disorder (for example, the user cannot continuously concentrate to an object), a conversion attention disorder (for example, if two target objects are generated, the user diverts attention to the next object before he/she completely takes one object), a divided attention disorder (for example, the user cannot take a target object outside the visual field where the space can be recognized (cannot see a notification image)), and a selective attention disorder (for example, the user cannot concentrate to an object as soon as a background is displayed (the user cannot discriminate an object that should be seen)) the user has.

### [Other Example Embodiments]

While the disclosure has been particularly shown and described with reference to example embodiments thereof, the invention is not limited to these example embodiments. It will be understood by those of ordinary skill in the art that various changes in form and details may be made therein, the scope of the present invention being defined by the claims.

The present disclosure is applicable to a system including a plurality of devices or a single apparatus. The present disclosure is also applicable even when an information processing program for implementing the functions of example embodiments is supplied to the system or apparatus directly or from a remote site. Hence, the present disclosure also incorporates the program installed in a computer to implement the functions of the present invention by the computer, a medium storing the program, and a WWW (World Wide Web) server that causes a user to download the program. Especially, the present disclosure incorporates at least a non-transitory computer readable medium storing a program that causes a computer to execute processing steps included in the above-described example embodiments.

## Claims

1. A cognitive ability estimation apparatus comprising:
an action detector (211) that is configured to detect a three-dimensional body action of a user;
a display controller (212) that is configured to generate and display, in a virtual space, an avatar object that moves in accordance with the detected body action and a target object for urging the user to perform the three-dimensional body action;
a setter (102) that is configured to set, as attributes of the target object in the virtual space, a moving speed, the number of displayed target objects, a size, a display position, and a display interval;
a feedback unit (214) that is configured to determine that the body action for the target object is achieved in case the avatar object hits the target object; and
an estimator (103) that is configured to estimate a cognitive ability level of the user in accordance with the attributes of the target object in a case where the body action for the target object can be achieved by the user or not,
**characterized in that**
the estimator (103) is configured to calculate the cognitive ability level using a value according to the moving speed of the target object in the virtual space, a value according to complexity of a background in the virtual space in which the target object is generated, a value according to a moving direction of the target object in the virtual space, a value according to the size of the target object in the virtual space, a value according to a size of an appearance range of the target object in the virtual space, a value according to whether the target object moves in a vertical direction, a value indicating a number of the target objects that simultaneously appear in the virtual space and an interval of appearance of the target object in the virtual space.

2. The cognitive ability estimation apparatus according to claim 1, wherein said presenter is configured to present the cognitive ability level estimated by said estimator, and present the attribute of the target object for which the body action should be achieved to improve a cognitive level.

3. The cognitive ability estimation apparatus according to claim 1, wherein said estimator is configured to estimate the cognitive ability level by subtracting a value according to the attribute of the target object from a reference value.

4. The cognitive ability estimation apparatus according to any one of claims 1 to 3, further comprising a storage unit that stores one of an equation and a table, each of which represent a relationship between a cognitive age and the attribute of the target object for which the user has achieved the body action,
wherein using one of the equation and the table, said estimator is configured to estimate the cognitive ability level of the user as the cognitive age.

5. The cognitive ability estimation apparatus according to any one of claims 1 to 4, wherein in accordance with the cognitive ability level of the user estimated by said estimator, said setter is configured to set the attribute of the target object to be displayed next.

6. The cognitive ability estimation apparatus according to any one of claims 1 to 5, wherein said estimator is further configured to estimate one of a driving risk and a falling risk based on the estimated cognitive ability level.

7. The cognitive ability estimation apparatus according to claim 6, wherein based on the estimated cognitive ability level, said setter is configured to propose a treatment program for improving cognitive ability, reducing the driving risk, or reducing the falling risk.

8. A cognitive ability estimation program for causing a computer to execute:
generating and displaying, in a virtual space, a target object for urging a user to perform a three-dimensional body action;
detecting the three-dimensional body action of the user;
generating and displaying, in the virtual space, an avatar object that moves in accordance with the detected body action;
setting, as attributes of the target object in the virtual space, a moving speed, the number of displayed target objects, a size, a display position, and a display interval;
determining that the body action for the target object is achieved in case the avatar object hits the target object; and
estimating a cognitive ability level of the user in accordance with the attributes of the target object in a case where the body action for the target object can be achieved by the user or not,
**characterized by**:
calculating the cognitive ability level using a value according to the moving speed of the target object in the virtual space, a value according to complexity of a background in the virtual space in which the target object is generated, a value according to a moving direction of the target object in the virtual space, a value according to the size of the target object in the virtual space, a value according to a size of an appearance range of the target object in the virtual space, a value according to whether the target object moves in a vertical direction, a value indicating a number of the target objects that simultaneously appear in the virtual space and an interval of appearance of the target object in the virtual space.

## Patentansprüche

1. Kognitive Fähigkeit-Schätzvorrichtung mit
einer Aktionserfassungseinrichtung (211), die zum Erfassen einer dreidimensionalen Körperaktion eines Benutzers eingerichtet ist,
einer Anzeigesteuereinrichtung (212), die zum Erzeugen und Anzeigen eines Avatarobjekts, das sich gemäß der erfassten Körperaktion bewegt, und eines Zielobjekts in einem virtuellen Raum zum Drängen des Benutzers zum Durchführen der dreidimensionalen Körperaktion eingerichtet ist,
einer Einstelleinrichtung (102), die zum Einstellen einer Bewegungsgeschwindigkeit, der Anzahl angezeigter Zielobjekte, einer Größe, einer Anzeigeposition und eines Anzeigeintervalls als Attribute des Zielobjekts in dem virtuellen Raum eingerichtet ist,
einer Rückkopplungseinheit (214), die zum Bestimmen, dass die Körperaktion für das Zielobjekt erzielt ist, wenn das Avatarobjekt das Zielobjekt trifft, eingerichtet ist, und
einer Schätzeinrichtung (103), die zum Schätzen eines kognitiven Fähigkeitsgrads des Benutzers gemäß den Attributen des Zielobjekts in einem Fall, in dem die Körperaktion für das Zielobjekt durch den Benutzer erzielt werden kann oder nicht, eingerichtet ist,
**dadurch gekennzeichnet, dass**
die Schätzeinrichtung (103) zum Berechnen des kognitiven Fähigkeitsgrads unter Verwendung eines der Bewegungsgeschwindigkeit des Zielobjekts in dem virtuellen Raum entsprechenden Werts, eines Werts, der einer Komplexität eines Hintergrunds in dem virtuellen Raum, in dem das Zielobjekt erzeugt wird, entspricht, eines einer Richtung des Zielobjekts in dem virtuellen Raum entsprechenden Werts, eines der Größe des Zielobjekts in dem virtuellen Raum entsprechenden Werts, eines einer Größe eines Erscheinungsbereichs des Zielobjekts in dem virtuellen Raum entsprechenden Werts, eines Werts, der dem entspricht, ob sich das Zielobjekt in einer vertikalen Richtung bewegt, eines Werts, der eine Anzahl der Zielobjekte, die in dem virtuellen Raum gleichzeitig erscheinen, und ein Intervall des Erscheinens des Zielobjekts in dem virtuellen Raum angibt.

2. Kognitive Fähigkeit-Schätzeinrichtung nach Anspruch 1,
wobei die Präsentationseinrichtung zum Präsentieren des durch die Schätzeinrichtung geschätzten kognitiven Fähigkeitsgrads und Präsentieren des Attributs des Zielobjekts, für welches die Körperaktion zu erzielen ist, zum Verbessern eines kognitiven Niveaus eingerichtet ist.

3. Kognitive Fähigkeit-Schätzvorrichtung nach Anspruch 1,
wobei die Schätzeinrichtung zum Schätzen des kognitiven Fähigkeitsgrads durch Subtrahieren eines dem Attribut des Zielobjekts entsprechenden Werts von einem Referenzwert eingerichtet ist.

4. Kognitive Fähigkeit-Schätzvorrichtung nach einem der Ansprüche 1 bis 3, ferner mit einer Speichereinheit, die eine Gleichung oder eine Tabelle speichert, die jeweils eine Beziehung zwischen einem kognitiven Alter und dem Attribut des Zielobjekts, für welches der Benutzer die Körperaktion erzielt hat, darstellen,
wobei die Schätzeinrichtung unter Verwendung der Gleichung oder der Tabelle zum Schätzen des kognitiven Fähigkeitsgrads des Benutzers als das kognitive Alter eingerichtet ist.

5. Kognitive Fähigkeit-Schätzvorrichtung nach einem der Ansprüche 1 bis 4, wobei die Einstelleinrichtung gemäß dem kognitiven Fähigkeitsgrad des Benutzers, der durch die Schätzeinrichtung geschätzt wird, zum Einstellen des Attributs des als nächstes anzuzeigenden Zielobjekts eingerichtet ist.

6. Kognitive Fähigkeit-Schätzvorrichtung nach einem der Ansprüche 1 bis 5, wobei die Schätzeinrichtung ferner zum Schätzen eines Fahrrisikos oder eines Sturzrisikos beruhend auf dem geschätzten kognitiven Fähigkeitsgrad eingerichtet ist.

7. Kognitive Fähigkeit-Schätzvorrichtung nach Anspruch 6, wobei die Einstelleinrichtung beruhend auf dem geschätzten kognitiven Fähigkeitsgrad zum Vorschlagen eines Behandlungsprogramms zum Verbessern der kognitiven Fähigkeit, Verringern des Fahrrisikos oder Verringern des Sturzrisikos eingerichtet ist.

8. Kognitive Fähigkeit-Schätzprogramm zum Veranlassen eines Computers zum Ausführen
eines Erzeugens und Anzeigens eines Zielobjekts in einem virtuellen Raum, um einen Benutzer zum Durchführen einer dreidimensionalen Körperaktion zu drängen,
eines Erfassens der dreidimensionalen Körperaktion des Benutzers,
eines Erzeugens und Anzeigens eines Avatarobjekts in dem virtuellen Raum, das sich gemäß der erfassten Körperaktion bewegt,
eines Einstellens der Anzahl angezeigter Zielobjekte, einer Größe, einer Anzeigeposition und eines Anzeigeintervalls als Attribute des Zielobjekts in dem virtuellen Raum,
eines Bestimmens, dass die Körperaktion für das Zielobjekt erzielt wird, wenn das Avatarobjekt das Zielobjekt trifft, und
eines Schätzens eines kognitiven Fähigkeitsgrads des Benutzers gemäß den Attributen des Zielobjekts in einem Fall, in dem die Körperaktion für das Zielobjekt durch den Benutzer erzielt werden kann oder nicht,
**gekennzeichnet durch**
ein Berechnen des kognitiven Fähigkeitsgrads unter Verwendung eines Werts, der der Bewegungsgeschwindigkeit des Zielobjekts in dem virtuellen Raum entspricht, eines Werts, der einer Komplexität eines Hintergrunds in dem virtuellen Raum entspricht, in dem das Zielobjekt erzeugt wird, eines Werts, der einer Bewegungsrichtung des Zielobjekts in dem virtuellen Raum entspricht, eines Werts, der der Größe des Zielobjekts in dem virtuellen Raum entspricht, eines Werts, der einer Größe eines Erscheinungsbereichs des Zielobjekts in dem virtuellen Raum entspricht, eines Werts, der dem entspricht, ob sich das Zielobjekt in einer vertikalen Richtung bewegt, eines Werts, der eine Anzahl der Zielobjekte, die in dem virtuellen Raum gleichzeitig erscheinen, und ein Intervall des Erscheinens des Zielobjekts in dem virtuellen Raum angibt.

## Revendications

1. Appareil d'estimation de la capacité cognitive comprenant :
un détecteur d'action (211) configuré pour détecter une action corporelle tridimensionnelle d'un utilisateur ;
un contrôleur d'affichage (212) configuré pour générer et afficher, dans un espace virtuel, un objet avatar qui se déplace conformément à l'action corporelle détectée et un objet cible pour inciter l'utilisateur à effectuer l'action corporelle tridimensionnelle ;
un dispositif de réglage (102) configuré pour régler, en tant qu'attributs de l'objet cible dans l'espace virtuel, une vitesse de déplacement, le nombre d'objets cibles affichés, une taille, une position d'affichage et un intervalle d'affichage ;
une unité de rétroaction (214) configurée pour déterminer que l'action corporelle pour l'objet cible est réalisée si l'objet avatar touche l'objet cible ; et
un estimateur (103) configuré pour estimer un niveau de la capacité cognitive de l'utilisateur en fonction des attributs de l'objet cible dans un cas où l'action corporelle pour l'objet cible peut être réalisée par l'utilisateur ou non,
**caractérisé en ce que**
l'estimateur (103) est configuré pour calculer le niveau de la capacité cognitive en utilisant une valeur en fonction de la vitesse de déplacement de l'objet cible dans l'espace virtuel, une valeur en fonction de la complexité d'un arrière-plan dans l'espace virtuel dans lequel l'objet cible est généré, une valeur en fonction d'une direction de déplacement de l'objet cible dans l'espace virtuel, une valeur en fonction de la taille de l'objet cible dans l'espace virtuel, une valeur en fonction d'une taille d'une plage d'apparition de l'objet cible dans l'espace virtuel, une valeur selon que l'objet cible se déplace ou non dans une direction verticale, une valeur indiquant un nombre d'objets cibles apparaissant simultanément dans l'espace virtuel et un intervalle d'apparition de l'objet cible dans l'espace virtuel.

2. Appareil d'estimation de la capacité cognitive selon la revendication 1, dans lequel ledit présentateur est configuré pour présenter le niveau de la capacité cognitive estimé par ledit estimateur, et présenter l'attribut de l'objet cible pour lequel l'action corporelle doit être réalisée afin d'améliorer un niveau cognitif.

3. Appareil d'estimation de la capacité cognitive selon la revendication 1, dans lequel ledit estimateur est configuré pour estimer le niveau de la capacité cognitive en soustrayant une valeur en fonction de l'attribut de l'objet cible d'une valeur de référence.

4. Appareil d'estimation de la capacité cognitive selon l'une quelconque des revendications 1 à 3 comprenant en outre une unité de stockage qui stocke un parmi une équation et un tableau, chacun représentant une relation entre un âge cognitif et l'attribut de l'objet cible pour lequel l'utilisateur a réalisé l'action corporelle,
dans lequel, en utilisant l'un parmi l'équation et le tableau, ledit estimateur est configuré pour estimer le niveau de la capacité cognitive de l'utilisateur en tant qu'âge cognitif.

5. Appareil d'estimation de la capacité cognitive selon l'une quelconque des revendications 1 à 4, dans lequel, en fonction du niveau de la capacité cognitive de l'utilisateur estimé par ledit estimateur, ledit dispositif de réglage est configuré pour régler l'attribut de l'objet cible à afficher ensuite.

6. Appareil d'estimation de la capacité cognitive selon l'une quelconque des revendications 1 à 5, dans lequel ledit estimateur est en outre configuré pour estimer un parmi un risque de conduite et un risque de chute sur la base du niveau de la capacité cognitive estimé.

7. Appareil d'estimation de la capacité cognitive selon la revendication 6, dans lequel, sur la base du niveau de la capacité cognitive estimé, ledit dispositif de réglage est configuré pour proposer un programme de traitement pour améliorer la capacité cognitive, réduire le risque de conduite ou réduire le risque de chute.

8. Programme d'estimation de la capacité cognitive destiné à faire exécuter un ordinateur de :
générer et afficher, dans un espace virtuel, un objet cible pour inciter un utilisateur à effectuer une action corporelle tridimensionnelle ;
détecter l'action corporelle tridimensionnelle de l'utilisateur ;
générer et afficher, dans l'espace virtuel, un objet avatar qui se déplace conformément à l'action corporelle détectée ;
régler, en tant qu'attributs de l'objet cible dans l'espace virtuel, une vitesse de déplacement, le nombre d'objets cibles affichés, une taille, une position d'affichage et un intervalle d'affichage ;
déterminer que l'action corporelle pour l'objet cible est réalisée si l'objet avatar touche l'objet cible ; et
estimer un niveau de la capacité cognitive de l'utilisateur en fonction des attributs de l'objet cible dans le cas où l'action corporelle pour l'objet cible peut être réalisée par l'utilisateur ou non,
**caractérisé par** :
calculer le niveau de la capacité cognitive en utilisant une valeur en fonction de la vitesse de déplacement de l'objet cible dans l'espace virtuel, une valeur en fonction de la complexité d'un arrière-plan dans l'espace virtuel dans lequel l'objet cible est généré, une valeur en fonction d'une direction de déplacement de l'objet cible dans l'espace virtuel, une valeur en fonction de la taille de l'objet cible dans l'espace virtuel, une valeur en fonction d'une taille d'une plage d'apparition de l'objet cible dans l'espace virtuel, une valeur selon que l'objet cible se déplace ou non dans une direction verticale, une valeur indiquant un nombre d'objets cibles apparaissant simultanément dans l'espace virtuel et un intervalle d'apparition de l'objet cible dans l'espace virtuel.
